(19) Europäisches Patentamt
European Patent Office
Office européen des brevets

(11) EP 3 604 279 B1

(12) EUROPEAN PATENT SPECIFICATION

(45) Date of publication and mention of the grant of the patent:
**16.08.2023 Bulletin 2023/33**

(21) Application number: **18770379.8**

(22) Date of filing: **12.03.2018**

(51) International Patent Classification (IPC):
***C07C 319/20*** *(2006.01)* ***C01B 17/36*** *(2006.01)*
***C07C 321/14*** *(2006.01)* ***C08G 18/38*** *(2006.01)*
***G02B 1/04*** *(2006.01)* ***G02C 7/00*** *(2006.01)*
***B29D 11/00*** *(2006.01)* ***C07C 319/14*** *(2006.01)*
***C07C 335/32*** *(2006.01)* ***C08G 18/00*** *(2006.01)*
***G02B 1/00*** *(2006.01)* ***C08G 18/24*** *(2006.01)*
***C08G 18/76*** *(2006.01)*

(52) Cooperative Patent Classification (CPC):
(C-Sets available)
**C07C 335/32; C07C 319/14; C07C 319/20; C08G 18/246; C08G 18/3876; C08G 18/7642; G02B 1/041;** B29D 11/00009 (Cont.)

(86) International application number:
**PCT/JP2018/009391**

(87) International publication number:
**WO 2018/173820 (27.09.2018 Gazette 2018/39)**

(54) **METHOD FOR PRODUCING POLYTHIOL COMPOUND, POLYMERIZABLE COMPOSITION AND USE THEREOF**

VERFAHREN ZUR HERSTELLUNG EINER POLYTHIOLVERBINDUNG, POLYMERISIERBARE ZUSAMMENSETZUNG UND DEREN VERWENDUNG

PROCÉDÉ DE PRODUCTION D'UN COMPOSÉ POLYTHIOL, COMPOSITION POLYMÉRISABLE ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2017 JP 2017058787**

(43) Date of publication of application:
**05.02.2020 Bulletin 2020/06**

(73) Proprietor: **Mitsui Chemicals, Inc.**
**Tokyo 104-0028 (JP)**

(72) Inventors:
- **TOKUNAGA, Koichi**
  **Omuta-shi**
  **Fukuoka 836-8610 (JP)**
- **KUMA, Shigetoshi**
  **Omuta-shi**
  **Fukuoka 836-8610 (JP)**
- **SHIMAKAWA, Chitoshi**
  **Omuta-shi**
  **Fukuoka 836-8610 (JP)**

(74) Representative: **Mewburn Ellis LLP**
**Aurora Building**
**Counterslip**
**Bristol BS1 6BX (GB)**

(56) References cited:
**EP-A1- 2 845 848**
**EP-A2- 0 300 859**
**WO-A1-2014/027428**
**JP-A- S6 428 207**
**JP-A- H07 252 207**
**JP-A- 2006 284 920**
**JP-B1- S 339 126**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 319/14, C07C 323/12;**
**C07C 319/20, C07C 321/14;**
**G02B 1/041, C08L 75/04, C08L 81/00**

## Description

TECHNICAL FIELD

The present invention relates to a process for producing a polythiol compound, a polymerizable composition, and uses thereof.

BACKGROUND ART

In comparison with inorganic lenses, plastic lenses are lighter, harder to break, and are able to be dyed and have thus rapidly become widespread in recent years as possible materials forming spectacle lenses, and camera lenses.

In recent years, there has been a demand for even higher performances for plastic lenses, specifically, there is a demand for a higher refractive index, a higher Abbe number, a lower specific gravity, and a higher heat resistance. So far, various resin materials for lenses have been developed and used.

Among these, an optical material formed of a polythiourethane-based resin has a high refractive index and a high Abbe number, and is excellent in impact resistance, dyeability, and processability. The polythiourethane-based resin is obtained by reacting a polythiol with a polyiso (thio) cyanate compound.

In cases where polythiourethane-based resins are used for plastic lenses, there is a demand for the polythiourethane-based resins to have less color, to be excellent in resin color, and to be transparent. In cases where the quality of the polythiol was low, the quality of the obtained resin was also low in some cases. Here, examples of patent documents relating to methods for manufacturing polythiol include the following.

Patent Documents 1 and 2 describe a method in which 2-mercaptoethanol and epichlorohydrin are reacted, the obtained compound is reacted with thiourea to obtain an isothiuronium salt, and then the isothiuronium salt is hydrolyzed to obtain a specific polythiol compound.

Patent Document 3 describes a process for producing a polythiol compound via a polyalcohol by reacting 2-mercaptoethanol with an epihalohydrin compound, in which 2-mercaptoethanol in which the content of the disulfide compound is 0.5% by weight or less is used.

Patent Document 4 describes a process for producing a (poly) thiol compound by reacting an organic (poly) halogen compound or (poly) alcohol compound with thiourea to form an isothiuronium salt, and hydrolyzing the obtained isothiuronium salt, in which thiourea in which the calcium content is 1.0% by weight or less is used.

Patent Document 5 describes methods for manufacturing a polythiol compound, including a method in which the calcium content included in thiourea and the amounts of specific impurities included in the 2-mercaptoethanol are set within a predetermined range.

Patent Document 6 describes methods for manufacturing pentaerythritol carboxylic acid ester, including a method in which the absorbance of a 5% by weight aqueous solution of pentaerythritol at a wavelength of 270 nm is set to a predetermined range.

Patent Document 7 describes methods for manufacturing a sulfur-containing monomer for an optical material, including a manufacturing method in which 50 mol% or more of a raw material is used in which a b* value of the raw material to be used is in a specific range. In this document, the measurement wavelength of the b* value is 400 to 700 nm in the visible light range.

Patent Document 8 describes a method for producing a polythiol compound comprising a step of reacting a polyalcohol with thiourea in the presence of hydrogen chloride, a step for adding aqueous ammonia, and a step for adding hydrochloric acid.

RELATED DOCUMENT

PATENT DOCUMENT

[Patent Document 1] JPH2-270859
[Patent Document 2] JPH7-252207
[Patent Document 3] WO2007/129449
[Patent Document 4] WO2007/129450
[Patent Document 5] KR10-2012-0058635
[Patent Document 6] WO2016/208707
[Patent Document 7] JP2006-284920
[Patent Document 8] EP 2845848

SUMMARY OF THE INVENTION

TECHNICAL PROBLEM

In a case where a plastic lens comprised of a polythiourethane-based resin is manufactured using a polythiol compound obtained by the methods described in these documents, it is possible to manufacture a plastic lens of good quality in terms of color, transparency, and striae.

However, in recent years, there has been a demand to further improve the appearance characteristics of optical members such as plastic lenses beyond the quality demanded in the related art.

Here, in order to improve the appearance characteristics of the optical member, for example, the addition of modifiers or additives or improvements such as adjustment of the polymerization conditions may be considered at the stage of obtaining a molded product by polymerization.

However, in these methods, there are concerns that other physical properties may be impaired by the addition of modifiers or additives, that the process efficiency may be reduced by adjusting the polymerization conditions.

From these reasons, it is considered as preferable to improve the raw material itself at the time of obtaining a molded product.

In view of such circumstances, it is an object of the present invention to provide a process for producing a polythiol compound capable of obtaining a colorless and transparent molded product which has a superior appearance in terms of color.

SOLUTION TO PROBLEM

As a result of intensive studies to solve the above problems, the present inventors found that the color of the obtained molded products is improved by controlling sodium sulfide as a raw material so as to satisfy a specific absorbance when obtaining a polythiol compound.

That is, the inventors found that it is possible to stably produce a colorless and transparent molded product which has an excellent appearance in terms of color by using this sodium sulfide, thereby completing the invention.

That is, it is possible to illustrate the present invention as follows.

The present invention provides a process for producing a polythiol compound, comprising:

reacting a compound represented by following General Formula (3) with sodium sulfide to obtain a polyalcohol compound represented by following Formula (4), and

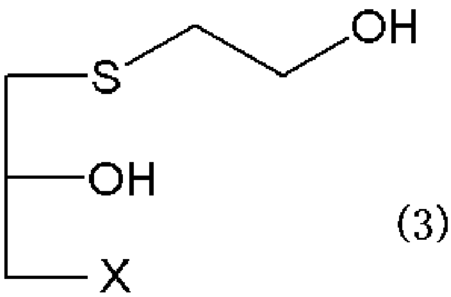

(3)

wherein, in the formula, X represents a halogen atom,

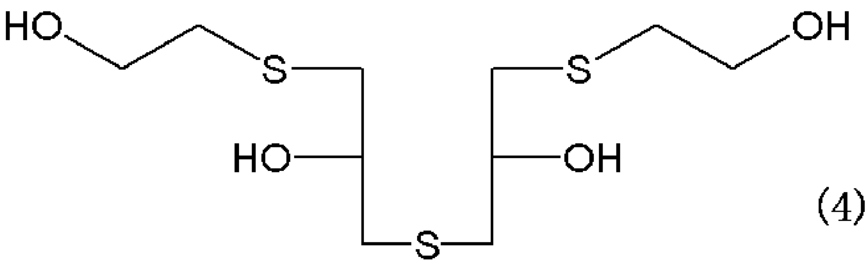

(4)

reacting the obtained polyalcohol compound represented by Formula (4) with a thiating agent to obtain a polythiol compound,

wherein an absorbance of 17.3% by weight aqueous solution of the sodium sulfide at a wavelength of 350 nm, which is measured with a quartz cell with an optical path length of 50 mm, is 0.70 or less. In some embodiments, the absorbance is 0.35 or less. In some embodiments, the process further comprises:

reacting 2-mercaptoethanol with an epihalohydrin compound represented by following General Formula (1) to obtain a compound represented by following General Formula (3) before obtaining the polyalcohol compound,

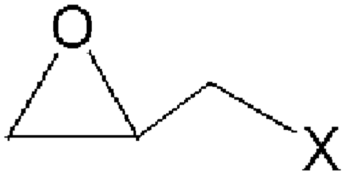

(1)

wherein, in the formula, X represents a halogen atom,

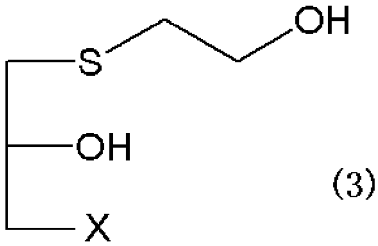

(3)

wherein, in the formula, X represents a halogen atom. In some embodiments, reacting the polyalcohol compound with a thiating agent includes

reacting the obtained polyalcohol compound represented by Formula (4) with a thiating agent in a presence of hydrogen chloride to obtain an isothiuronium salt, and

adding ammonia to a reaction solution including the obtained isothiuronium salt and hydrolyzing the isothiuronium salt to obtain a polythiol compound having at least one kind selected from the group consisting of compounds represented by following Formulae (6) to (8) as a main component.

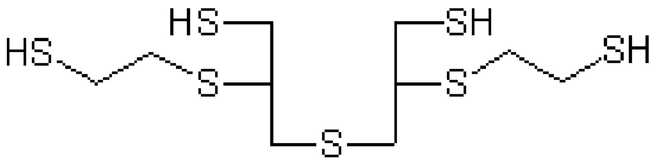

(6)

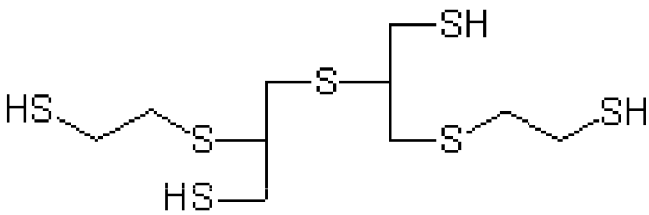

(7)

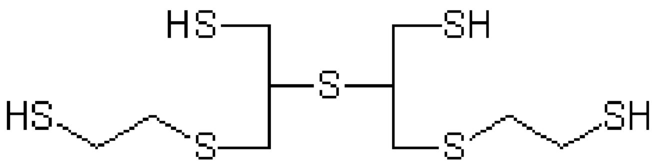

(8)

In some embodiments, the thiating agent is thiourea.

In some embodiments, the absorbance is 0.003 or more.

In some embodiments, X in the General Formula is a chlorine atom. In some embodiments, the process further

comprises adding hydrogen chloride to the solution including the obtained polythiol compound and washing to purify the polythiol compound, after reacting the polyalcohol compound with a thiating agent.

The invention also provides a polymerizable composition comprising:

the polythiol compound obtained by the manufacturing method described above and
a polyiso(thio)cyanate compound.

The invention also provides a resin obtained by curing the polymerizable composition described above.

The invention also provides an optical material comprising:
the resin described above.

The invention also provides a lens comprising:
the resin described above.

The process for producing a polythiol compound may further comprise prior to the step of reacting the compound represented by General Formula (3) with sodium sulfide, preparing 17.3% by weight aqueous solution of the sodium sulfide and measuring an absorbance at a wavelength of 350 nm of the aqueous solution with a quartz cell with an optical path length of 50 mm to screening the sodium sulfide that the measured absorbance of the aqueous solution is 0.70 or less.

In some embodiments, the measured absorbance of the aqueous solution is 0.35 or less.

ADVANTAGEOUS EFFECTS OF INVENTION

The process for producing a polythiol compound of the present invention can improve the color of the polythiol compound by using sodium sulfide having an absorbance within a specific range, and further improve the appearance of the color of molded products obtained from the polythiol compound.

DESCRIPTION OF EMBODIMENTS

Based on the following embodiments, a description will be given of "a process for producing a polythiol compound which has at least one kind selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane as a main component (referred to below simply as the polythiol compound) " according to the present invention.

[Process for producing Polythiol Compound]

The process for producing the polythiol compound according to the present embodiment includes steps (A) to (E) described below, and sodium sulfide used in step (B) described below has an absorbance at a wavelength of 350 nm of 0.70 or less in 17.3% by weight aqueous solution thereof, as measured with a quartz cell with an optical path length of 50 mm.

That is, in the process for producing a polythiol compound according to the present embodiment, in a case where 17.3% by weight aqueous solution of sodium sulfide is prepared and the absorbance is measured with a quartz cell with an optical path length of 50 mm, sodium sulfide in which the absorbance at a wavelength of 350 nm is a specific value is used as the raw material.

A description will be given below of the sodium sulfide used in the process for producing the polythiol compound according to the present embodiment.

(Sodium Sulfide)

In the present embodiment, a sodium sulfide is used which has an absorbance at a wavelength of 350 nm of 0.70 or less in a case where 17.3% by weight aqueous solution thereof is prepared and the aqueous solution is measured with a quartz cell with an optical path length of 50 mm.

The aqueous sodium sulfide solution satisfying the above values makes it possible to provide an excellent color in the polythiol compound produced using this aqueous sodium sulfide solution, and to further improve the appearance of the color of a thiourethane molded product obtained by further reacting the polythiol compound with the polyiso(thio)cyanate compound.

Here, the 350 nm wavelength is not a wavelength corresponding to the so-called visible region, and the substance itself which contributes to the value of the absorbance at the 350 nm wavelength does not bring about changes in the optical characteristics of the molded product in the visible region. However, the present inventors found that

substances contributing to the value of the absorbance at the 350 nm wavelength bring about some interactions when obtaining a resin by polymerization and influence the optical characteristics of the obtained molded product. It is also possible to describe the sodium sulfide in the present embodiment as a sodium sulfide-containing composition including the above substance or a sodium sulfide material including the above substance.

That is, the present inventors found that controlling the sodium sulfide used as a raw material so as to satisfy the above value is an important matter in terms of securing the quality of the sodium sulfide used as a raw material and, in addition, found that it is possible to solve the problems of the present application.

In addition, for the sodium sulfide used in the process for producing a polythiol compound according to the present embodiment, the absorbance of the 17.3% by weight aqueous sodium sulfide solution at a wavelength of 350 nm is preferably 0.70 or less, more preferably, in order, 0.50 or less, 0.35 or less, 0.34 or less, and 0.33 or less, and particularly preferably 0.325 or less.

Satisfying such a value makes it possible to further improve the appearance of the color of the obtained molded product.

Here, it is possible to adopt the following conditions in the measurement of the absorbance at the wavelength of 350 nm.

First, 17.3% by weight aqueous solution is prepared from sodium sulfide, and this aqueous solution is added into a quartz cell with an optical path length of 50 mm to determine the absorbance at a wavelength of 350 nm.

For the measurement of the absorbance, it is possible to use a spectrophotometer manufactured by Shimadzu Corporation (equipment name: UV-1600).

In addition, for the sodium sulfide used in the process for producing a polythiol compound according to the present embodiment, the absorbance of the 17.3% by weight aqueous sodium sulfide solution at a wavelength of 350 nm is preferably 0.003 or more, more preferably 0.005 or more, and even more preferably 0.01 or more.

In a case where the absorbance of 17.3% by weight aqueous solution of sodium sulfide at a wavelength of 350 nm is the above lower limit or more, excess purification is unnecessary, and it is possible to improve the process efficiency as a whole. In this manner, improving the process efficiency as a whole makes it possible to improve the productivity of the polythiol compound excellent in appearance and and the productivity of the resin using the polythiol compound.

In the present embodiment, in a case where the absorbance of sodium sulfide to be used is outside the range of the present invention in the measurement method described above or in a case where the absorbance of sodium sulfide after time passes is outside the range of the present invention, it is possible to adjust the absorbance of the 17.3% by weight aqueous sodium sulfide solution at a wavelength of 350 nm into the range described above by purifying the sodium sulfide.

Examples of purification methods include a method for dissolving sodium sulfide in a solvent and then carrying out purification by crystallization. Water is usually used as the solvent. Distilled water or ion exchange water may be used as the water to be used as a solvent. In addition, before the sodium sulfide is crystallized from the solution in which sodium sulfide is dissolved, the sodium sulfide solution may be treated using a specific treatment agent and then the sodium sulfide may be crystallized. Examples of treatment agents include various adsorbents such as activated carbon, activated alumina, silica gel, and a molecular sieve, ion exchange resins, and chelate resins. The crystallization may be repeated a plurality of times, or may be performed a plurality of times by changing the treatment agent.

The absorbance of commercially available sodium sulfide may be within the range of the present invention in the above measurement method or may be outside this range. In such a case, it is also possible to select and use sodium sulfide which is within the absorbance range in the present invention.

Next, an explanation will be given of the process for producing the "polythiol compound having, as a main component, at least one kind selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane" which is the polythiol compound of the present invention.

The process for producing the polythiol compound of the present invention includes the following steps.

Step I: A compound represented by following General Formula (3) is reacted with sodium sulfide to obtain a polyalcohol compound represented by following Formula (4).

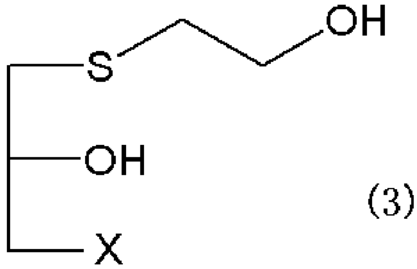

(3)

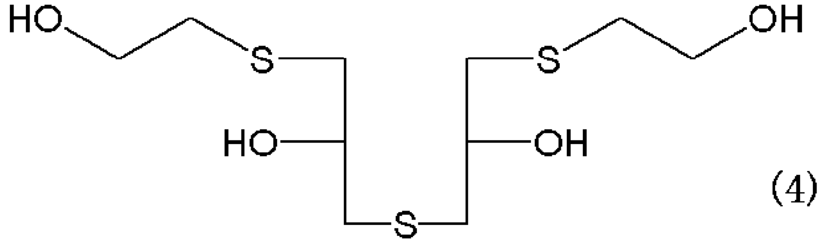

(4)

In Formula (3), X represents a halogen atom.

Step II: The obtained polyalcohol compound represented by the Formula (4) is reacted with a thiating agent to obtain a polythiol compound.

Examples of thiating agents include thiourea, thiocyanates such as sodium thiocyanate, potassium thiocyanate, ammonium thiocyanate, calcium thiocyanate, and lead thiocyanate.

In the present embodiment, a description will be given of the process for producing the polythiol compound of the present invention using the following steps (A) to (E).

Step A: 2-mercaptoethanol is reacted with an epihalohydrin compound represented by following General Formula (1) to obtain a compound represented by following General Formula (3).

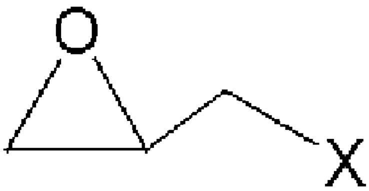

(1)

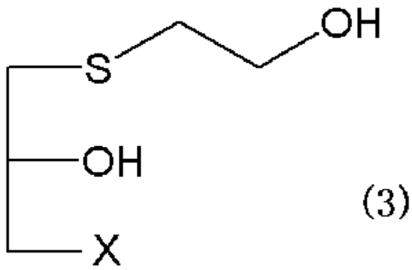

(3)

In Formulae (1) or (3), X represents a halogen atom.

Step B: The compound represented by General Formula (3) obtained in step A is reacted with sodium sulfide to obtain a polyalcohol compound represented by following Formula (4).

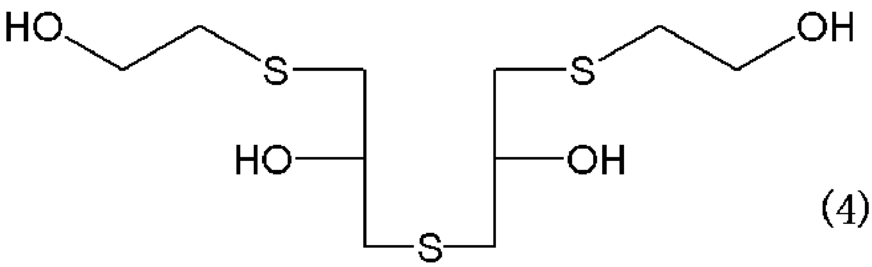

(4)

Step C: The polyalcohol compound represented by Formula (4) obtained in step B and thiourea are reacted in the presence of hydrogen chloride to obtain an isothiuronium salt.

Step D: A base compound, for example, ammonia, is added to a reaction solution including the isothiuronium salt obtained in step C, the isothiuronium salt is hydrolyzed to obtain a polythiol compound having, as a main component, at least one kind selected from the group consisting of compounds represented by following Formulae (6) to (8) .

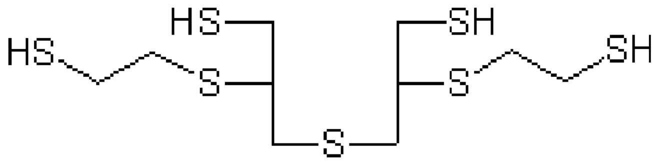

(6)

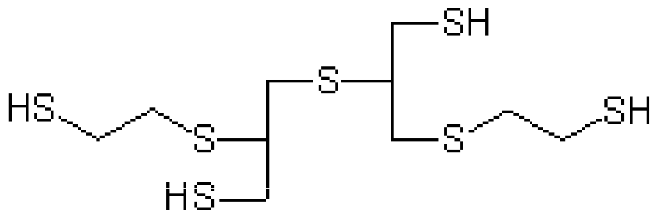

(7)

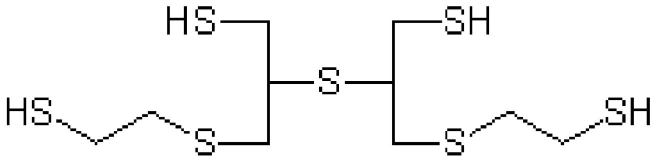

(8)

Step E: Hydrogen chloride is added to a solution including the polythiol compound obtained in step D and washed to purify the polythiol compound.
A description will be given below of each step in order.

(Step A)

In the present embodiment, first, it is possible to obtain a diol compound represented by following General Formula (3) by reacting 2-mercaptoethanol with an epihalohydrin compound represented by following General Formula (1).

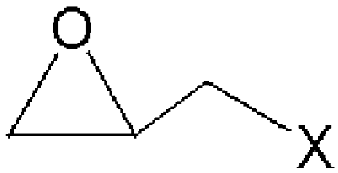

(1)

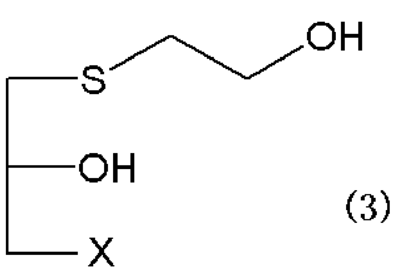

(3)

In General Formula (1) or (3), X is a halogen atom which is a fluorine atom, a chlorine atom, a bromine atom, or an iodine atom, and preferably a chlorine atom.
In the present embodiment, it is possible to perform the reaction in a range of 2 to 30°C. It is possible to perform the reaction in 2 to 10 hours.
It is possible to perform the reaction specifically as follows.
First, epihalohydrin is added dropwise to 2-mercaptoethanol, to an aqueous solution of 2-mercaptoethanol as necessary, to an aqueous solution of 2-mercaptoethanol containing a lower alcohol such as methanol and ethanol and a base compound, or to a lower alcohol solution such as methanol or ethanol containing 2-mercaptoethanol. The reaction

temperature and reaction time are preferably adjusted to be in the range described above.

Examples of base compounds include metal hydroxides such as sodium hydroxide and potassium hydroxide, metal carbonates such as sodium carbonate and potassium carbonate, and tertiary amines such as triethylamine and tributylamine; however, sodium hydroxide and ammonia are preferable in terms of reactivity and economy.

In the solution into which the epihalohydrin was added dropwise, the amount of 2-mercaptoethanol used is approximately 0.5 moles or more and 3 moles or less per mole of the epihalohydrin. It is possible to use the base compound as an aqueous solution, or an alcohol solution, and in a case of being used as a solution, it is possible to appropriately select the concentration of the base compound. By adding the epihalohydrin dropwise to the solution described above, a diol compound represented by General Formula (3) is obtained.

(Step B)

Next, it is possible to obtain the tetraol compound represented by Formula (4) by reacting the diol compound represented by General Formula (3) obtained in step A with sodium sulfide.

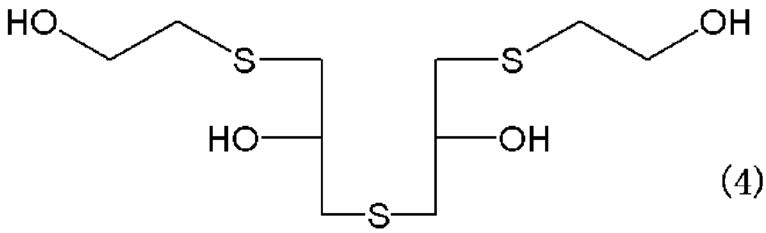

(4)

In the present embodiment, it is possible to perform the reaction at 10 to 50°C for 1 to 10 hours.

It is possible to perform the reaction specifically as follows.

An aqueous solution of sodium sulfide is added dropwise to a reaction solution including the diol compound after the reaction described above, or solid sodium sulfide is added thereto. The reaction temperature and reaction time are preferably adjusted to be in the ranges described above. It is possible to use the sodium sulfide in an amount of 0.4 mol to 0.6 mol per mole of the diol compound, preferably 0.45 mol to 0.57 mol, and more preferably 0.48 mol to 0.55 mol.

As the sodium sulfide used in step B, it is possible to use sodium sulfide for which, in a case of preparing 17.3% by weight aqueous solution thereof and measuring the aqueous solution using a quartz cell with an optical path length of 50 mm as described above, the absorbance at a wavelength of 350 nm was 0.70 or less.

(Step C)

Next, the tetraol compound represented by Formula (4) obtained in step B and thiourea are reacted in the presence of hydrogen chloride to obtain an isothiuronium salt.

Using hydrogen chloride makes it possible to obtain a sufficient reaction rate and control the coloration of the product. As hydrogen chloride, it is possible to use hydrochloric acid or hydrogen chloride gas.

(Step D)

A base compound, for example, an inorganic base such as sodium hydroxide, potassium hydroxide, potassium carbonate, sodium carbonate, sodium acetate, or potassium phosphate, or an organic base such as ammonia, triethylamine, or hydrazine are added to a reaction solution including the isothiuronium salt obtained in step C, the isothiuronium salt is hydrolyzed, and a polythiol compound is obtained. In a case where ammonia is used as the base compound, examples of ammonia include ammonia water or ammonia gas. In the present embodiment, a description will be given of an example using ammonia water.

In the present embodiment, as the polythiol compound, a polythiol compound having, as a main component, at least one kind selected from the group consisting of 4,8-dimercaptomethyl-1,11-dimercapto-3,6,9-trithiaundecane represented by following Formula (6), 4,7-dimercaptomethyl-1,11-dimercapto-3,6,9-trithiaundecane represented by following Formula (7), and 5,7-dimercaptomethyl-1,11-dimercapto-3,6,9-trithiaundecane represented by following Formula (8) is obtained.

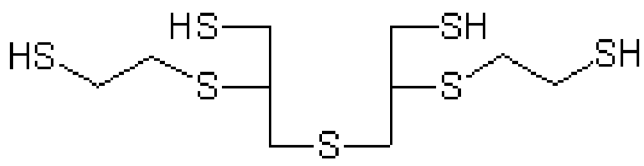

(6)

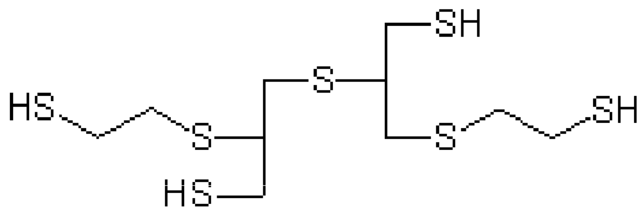

(7)

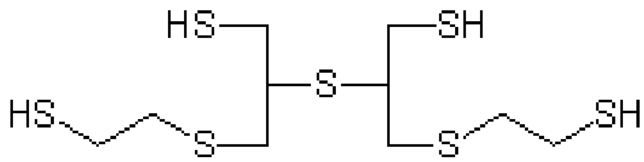

(8)

Specifically, while maintaining a reaction solution including the isothiuronium salt in a temperature range of 20 to 60°C, ammonia water is added to the reaction solution. The time during which the ammonia water is added is preferably short.

It is preferable to add an organic solvent before adding the ammonia water. Adding an organic solvent makes it possible to suppress the formation of by-products. The added amount of the organic solvent is appropriately selected according to the type of the solvent. Examples of organic solvents include toluene, xylene, chlorobenzene, and dichlorobenzene,. From the viewpoint of the above effects, toluene is preferable.

In a case where ammonia gas is added instead of part or all of the ammonia water, it is possible to perform the addition under the same conditions (use amount, addition time, addition temperature) as the ammonia water.

Then, after adding ammonia water, the hydrolysis reaction is continuously performed between room temperature and the reflux temperature range.

(Step E)

In the present embodiment, the polythiol compound obtained in step D is purified.

Specifically, it is possible to perform acid washing and then a plurality of water washings. It is also possible to perform water washing before the acid washing and alkali washing after the acid washing. It is possible for the alkali washing to reduce the number of times of water washing. It is possible to efficiently remove impurities by a washing step. Examples of preferable embodiments after hydrolysis include a method in which water washing, acid washing, water washing, alkali washing, and then water washing are performed, a method in which acid washing, water washing, alkali washing, and water washing are performed, a method in which acid washing and water washing are performed,. Each washing may be repeated a plurality of times.

It is possible to perform the acid washing by adding hydrogen chloride to a solution including the obtained polythiol compound. Examples of hydrogen chloride include hydrochloric acid, and hydrogen chloride gas,.

For water washing, it is possible to use degassed water having an oxygen concentration of 5 mg/L or less.

In addition, it is possible to perform the alkaline washing using an alkaline aqueous solution. Ammonia water is preferable as the alkaline aqueous solution.

After step E, a solvent removal step, and as necessary, a removal step of a low boiling point compound, a filtration step, and a distillation step are performed and it is possible to obtain a polythiol compound having, as a main component, at least one kind selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane, as the polythiol compound.

The solvent removal step is a step of removing the organic solvent under normal pressure or reduced pressure, and the degree of reduced pressure and temperature are appropriately selected depending on the solvent to be used.

It is possible to represent the color of the polythiol compound obtained by the manufacturing method of the

present embodiment using APHA. The color (APHA) of the polythiol compound is 20 or less, and the color is excellent.

[Polymerizable Composition]

The polymerizable composition of the present embodiment includes the polythiol compound obtained by the manufacturing method described above and a polyiso(thio)cyanate compound.

In addition, a "polyiso(thio)cyanate compound" means a polyisocyanate compound or a polyisothiocyanate compound.

Examples of polyiso(thio)cyanate compounds include compounds selected from the group consisting of aliphatic polyisocyanate compounds, alicyclic polyisocyanate compounds, aromatic polyisocyanate compounds, heterocyclic polyisocyanate compounds, aliphatic polyisothiocyanate compounds, alicyclic polyisothiocyanate compounds, aromatic polyisothiocyanate compounds, and sulfur-containing heterocyclic polyisothiocyanate compounds, and modified products thereof.

More specific examples include aliphatic polyisocyanate compounds such as pentamethylene diisocyanate, hexamethylene diisocyanate, 2,2,4-trimethylhexane diisocyanate, 2,4,4-trimethylhexamethylene diisocyanate, lysine diisocyanato methyl ester, lysine triisocyanate, m-xylylene diisocyanate, p-xylene diisocyanate, α, α, α', α'-tetramethyl xylylene diisocyanate, bis(isocyanatomethyl) naphthalene, mesitylene triisocyanate, bis(isocyanatomethyl) sulfide, bis(isocyanatoethyl) sulfide, bis(isocyanatomethyl) disulfide, bis(isocyanatoethyl) disulfide, bis(isocyanatomethylthio) methane, bis(isocyanatoethylthio) methane, bis(isocyanatoethylthio) ethane, bis(isocyanatomethylthio) ethane;

alicyclic polyisocyanate compounds such as isophorone diisocyanate, bis(isocyanatomethyl) cyclohexane, dicyclohexylmethane-4,4'-diisocyanate, cyclohexane diisocyanate, methylcyclohexane diisocyanate, dicyclohexyldimethylmethane isocyanate, 2,5-bis(isocyanatomethyl) bicyclo-[2.2.1]-heptane, 2,6-bis(isocyanatomethyl) bicyclo-[2.2.1]-heptane, 3,8-bis(isocyanatomethyl) tricyclodecane, 3,9-bis(isocyanatomethyl) tricyclodecane, 4,8-bis(isocyanatomethyl) tricyclodecane, and 4,9-bis(isocyanatomethyl) tricyclodecane;
aromatic polyisocyanate compounds such as phenylene diisocyanate, 2,4-tolylene diisocyanate, 2,6-tolylene diisocyanate, 4,4'-diphenylmethane diisocyanate, and diphenyl sulfide-4,4-diisocyanate;
heterocyclic polyisocyanate compounds such as 2,5-diisocyanatothiophene, 2,5-bis(isocyanatomethyl) thiophene, 2,5-diisocyanatotetrahydrothiophene, 2,5-bis(isocyanatomethyl) tetrahydrothiophene, 3,4-bis(isocyanatomethyl) tetrahydrothiophene, 2,5-diisocyanato-1,4-dithiane, 2,5-bis(isocyanatomethyl)-1,4-dithiane, 4,5-diisocyanato-1,3-dithiolane, and 4,5-bis(isocyanatomethyl)-1,3-dithiolane;. As a polyiso(thio)cyanate compound, it is possible to use at least one kind selected from the above in combination.

In addition, it is also possible to use halogen-substituted compounds such as chlorine-substituted compounds and bromine-substituted compounds, alkyl-substituted compounds, alkoxy-substituted compounds, nitro-substituted compounds, prepolymer-modified compounds of polyhydric alcohols, carbodiimide-modified compounds, urea-modified compounds, burette-modified compounds, and dimerization or trimerization reaction products, of the above compounds.

Examples of polyisothiocyanate compounds include aliphatic polyisothiocyanate compounds such as hexamethylene diisothiocyanate, lysine diisothiocyanate methyl ester, lysine triisothiocyanate, m-xylylene diisothiocyanate, bis(isothiocyanatomethyl) sulfide, bis(isothiocyanatoethyl) sulfide, and bis(isothiocyanatoethyl) disulfide;

alicyclic polyisothiocyanate compounds such as isophorone diisothiocyanate, bis(isothiocyanatomethyl) cyclohexane, dicyclohexylmethane diisothiocyanate, cyclohexane diisothiocyanate, methyl cyclohexane diisothiocyanate, 2,5-bis(isothiocyanatomethyl) bicyclo-[2.2.1]-heptane, 2,6-bis(isothiocyanatomethyl) bicyclo-[2.2.1]-heptane, 3,8-bis(isothiocyanatomethyl) tricyclodecane, 3,9-bis(isothiocyanatomethyl) tricyclodecane, 4,8-bis(isothiocyanatomethyl) tricyclodecane, and 4,9-bis(isothiocyanatomethyl) tricyclodecane;
aromatic polyisothiocyanate compounds such as tolylene diisothiocyanate, 4,4-diphenylmethane diisothiocyanate, and diphenyl disulfide-4,4-diisothiocyanate;
sulfur-containing heterocyclic polyisothiocyanate compounds such as 2,5-diisothiocyanatothiophene, 2,5-bis(isothiocyanatomethyl) thiophene, 2,5-isothiocyanatotetrahydrothiophene, 2,5-bis(isothiocyanatomethyl) tetrahydrothiophene, 3,4-bis(isothiocyanatomethyl) tetrahydrothiophene, 2,5-diisothiocyanato-1,4-dithiane, 2,5-bis(isothiocyanatomethyl)-1,4-dithiane, 4,5-diisothiocyanato-1,3-dithiolane, and 4,5-bis(isothiocyanatomethyl)-1,3-dithiolane;. As a polyiso(thio)cyanate compound, it is possible to use at least one kind selected from the above in combination.

In addition, it is also possible to use halogen-substituted compounds such as chlorine-substituted compounds and bromine-substituted compounds, alkyl-substituted compounds, alkoxy-substituted compounds, nitro-substituted compounds, prepolymer-modified products of polyhydric alcohols, carbodiimide-modified compounds, urea-modified compounds, burette-modified compounds, and dimerization or trimerization reaction products, of the above compounds.

In the present embodiment, as the polyiso (thio) cyanate compound, it is preferable to use at least one type selected from hexamethylene diisocyanate, pentamethylene diisocyanate, m-xylylene diisocyanate, isophorone diisocyanate, bis(isocyanatomethyl) cyclohexane, bis(isocyanatocyclohexyl) methane, 2,5-bis(isocyanatomethyl) bicyclo-[2.2.1]-heptane, 2,6-bis(isocyanatomethyl) bicyclo-[2.2.1]-heptane, tolylene diisocyanate, phenylene diisocyanate, and 4,4'-diphenylmethane diisocyanate.

In addition to the polythiol compound obtained by the method described above, it is also possible to use other polythiol compounds as the polythiol compound used for the polymerizable composition.

Examples of other polythiol compounds include aliphatic polythiol compounds such as methanedithiol, 1,2-ethanedithiol, 1,2,3-propanetrithiol, 1,2-cyclohexanedithiol, bis(2-mercaptoethyl) ether, tetrakis(mercaptomethyl) methane, diethylene glycol bis(2-mercaptoacetate), diethylene glycol bis(3-mercaptopropionate), ethylene glycol bis(2-mercaptoacetate), ethylene glycol bis(3-mercaptopropionate), trimethylolpropane tris (2-mercaptoacetate), trimethylolpropane tris (3-mercaptopropionate), trimethylol ethane tris (2-mercaptoacetate), trimethylol ethane tris (3-mercaptopropionate), pentaerythritol tetrakis(2-mercaptoacetate), pentaerythritol tetrakis(3-mercaptopropionate), bis(mercaptomethyl) sulfide, bis(mercaptomethyl) disulfide, bis(mercaptoethyl) sulfide, bis(mercaptoethyl) disulfide, bis(mercaptopropyl) sulfide, bis(mercaptomethylthio) methane, bis(2-mercaptoethylthio) methane, bis(3-mercaptopropylthio) methane, 1,2-bis(mercaptomethylthio) ethane, 1,2-bis(2-mercaptoethyl) thio) ethane, 1,2-bis(3-mercaptopropylthio) ethane, 1,2,3-tris (mercaptomethylthio) propane, 1,2,3-tris (2-mercaptoethylthio) propane, 1,2,3-tris (3-mercaptopropylthio) propane, 4-mercaptomethyl-1,8-dimercapto-3,6-dithiaoctane, tetrakis(mercaptomethylthiomethyl) methane, tetrakis(2-mercaptoethylthiomethyl) methane, tetrakis(3-mercaptopropylthiomethyl) methane, bis(2,3-dimercaptopropyl) sulfide, 2,5-dimercaptomethyl-1,4-dithiane, 2,5-dimercapto-1,4-dithiane, 2,5-dimercaptomethyl-2,5-dimethyl-1,4-dithiane, and thioglycolic acid and mercaptopropionic acid esters thereof, hydroxymethyl sulfide bis(2-mercaptoacetate), hydroxymethyl sulfide bis(3-mercaptopropionate), hydroxyethyl sulfide bis(2-mercaptoacetate), hydroxyethyl sulfide bis(3-mercapto propionate), hydroxymethyl disulfide bis(2-mercaptoacetate), hydroxymethyl disulfide bis(3-mercaptopropionate), hydroxyethyl disulfide bis(2-mercaptoacetate), hydroxyethyl disulfide bis(3-mercaptopropionate), 2-mercaptoethyl ether bis(2-mercaptoacetate), 2-mercaptoethyl ether bis(3-mercaptopropionate), thiodiglycolic acid bis (2-mercaptoethyl ester), thiodipropionic acid bis(2-mercaptoethyl ester), dithiodiglycolic acid bis(2-mercaptoethyl ester), dithiodipropionic acid bis(2-mercaptoethyl ester), 1,1,3,3-tetrakis(mercaptomethylthio) propane, 1,1,2,2-tetrakis(mercaptomethylthio) ethane, 4,6-bis(mercaptomethylthio)-1,3-dithiane, tris (mercaptomethylthio) methane, and tris (mercaptoethylthio) methane;

aromatic polythiol compounds such as 1,2-dimercaptobenzene, 1,3-dimercaptobenzene, 1,4-dimercaptobenzene, 1,2-bis(mercaptomethyl) benzene, 1,3-bis(mercaptomethyl) benzene, 1,4-bis(mercaptomethyl) benzene, 1,2-bis(mercaptoethyl) benzene, 1,3-bis(mercaptoethyl) benzene, 1,4-bis(mercaptoethyl) benzene, 1,3,5-trimercaptobenzene, 1,3,5-tris (mercaptomethyl) benzene, 1,3,5-tris (mercaptomethyleneoxy) benzene, 1,3,5-tris (mercaptoethyleneoxy) benzene, 2,5-toluenedithiol, 3,4-toluenedithiol, 1,5-naphthalenedithiol, and 2,6-naphthalenedithiol;

heterocyclic polythiol compounds such as 2-methylamino-4,6-dithiol-sym-triazine, 3,4-thiophenedithiol, bismuthiol, and 2-(2,2-bis(mercaptomethylthio) ethyl)-1,3-dithietane.

However, the other polythiol compounds are not limited to these exemplified compounds. In the present embodiment, it is also possible to use at least one type selected from the above in combination.

Other examples of preferable polythiol compounds include aliphatic polythiol compounds such as methanedithiol, 1,2-ethanedithiol, 1,2,3-propanetrithiol, pentaerythritol tetrakis(2-mercaptoacetate), pentaerythritol tetrakis(3-mercaptopropionate), bis(mercaptoethyl) sulfide, 4-mercaptomethyl-1,8-dimercapto-3,6-dithiaoctane, 2,5-dimercaptomethyl-1,4-dithiane, tetrakis(mercaptomethylthiomethyl) methane, tetrakis(2-mercaptoethylthiomethyl) methane, tetrakis(3-mercaptopropylthiomethyl) methane, bis(2,3-dimercaptopropyl) sulfide, 2,5-dimercapto-1,4-dithiane, 2,5-dimercaptomethyl-2,5-dimethyl-1,4-dithiane, 1,1,3,3-tetrakis(mercaptomethylthio) propane, 1,1,2,2-tetrakis(mercaptomethylthio) ethane, 4,6-bis(mercaptomethylthio)-1,3-dithiane.

In addition to the polythiol compounds described above, a polyol compound may be included.

In addition to the polythiol compound and the polyiso(thio)cyanate compound forming the polythiourethane-based resin, other substances may be added for the purpose of improving the various physical properties, operability, and polymerization reactivity, of the polythiourethane-based resin of the present embodiment. For example, in addition to the urethane-forming raw material, at least one kind of active hydrogen compounds represented by amines, carbonate compounds, ester compounds, metals, metal oxides, organic metal compounds, and inorganic substances may be added.

Furthermore, for the purpose of modifying the resin, a resin modifier such as an olefin compound including a hydroxy compound, an epoxy compound, or an episulfide compound, an organic acid and an anhydride thereof, a (meth) acrylate compound, may be added. Here, the resin modifier is a compound which adjusts or improves physical properties such as the refractive index, Abbe number, heat resistance, and specific gravity, mechanical strength such as impact resistance, of the material comprised of the thiourethane-based resin.

In addition, depending on the purpose, similar to known molding methods, various substances such as chain extenders, cross-linking agents, light stabilizers, UV absorbers, antioxidants, internal release agents, bluing agents, oil-soluble dyes, and fillers may be added.

In addition, in order to adjust to a desired reaction rate, a well-known reaction catalyst used in the manufacturing of polythiourethane-based resins may be added as appropriate.

Examples of reaction catalysts include dialkyl tin halides such as dibutyl tin dichloride and dimethyl tin dichloride, dialkyl tin dicarboxylates such as dimethyl tin diacetate, dibutyl tin dioctanoate, and dibutyl tin dilaurate, dialkyl tin dialkoxides such as dibutyl tin dibutoxide and dioctyl tin dibutoxide; dialkyl tin dithioalkoxides such as dibutyl tin di (thiobutoxide); dialkyl tin oxides such as di (2-ethylhexyl) tin oxide, dioctyl tin oxide, and bis(butoxy dibutyl tin) oxide; dialkyl tin sulfides such as dibutyl tin sulfide, and thiocarbamic acid S-alkyl ester. Among the above, preferable examples include dialkyl tin halides such as dibutyl tin dichloride and dimethyl tin dichloride, and thiocarbamic acid S-alkyl esters.

[Resin]

It is possible to obtain a resin by curing the polymerizable composition including the polythiol compound obtained as described above and the polyiso(thio)cyanate compound, and other polythiol compounds, additives, and modifiers included as necessary.

The use ratio of the polythiol compound and the polyiso(thio)cyanate compound is not particularly limited, but the molar ratio is usually in the range of SH group/NCO group = 0.5 to 3.0, preferably 0.6 to 2.0, and more preferably in the range of 0.8 to 1.3. When the use ratio is in the ranges described above, it is possible to satisfy various properties such as the refractive index and heat resistance demanded as an optical material such as a plastic lens and a transparent material, in a well-balanced manner.

Specifically, the polymerizable composition of the present embodiment is obtained as a mixed liquid by mixing the polythiol compound obtained by the manufacturing method described above, the polyiso(thio)cyanate compound, and other components as necessary. It is possible to obtain a molded product by degassing this mixed liquid by an appropriate method as necessary, then pouring the result into a mold, and normally carrying out gradual heating from a low temperature to a high temperature, and polymerization.

The molded product comprised of the resin of the present embodiment obtained in this manner has a characteristic of an excellent color and is further colorless and transparent, with the generation of white turbidity being suppressed. It is possible to represent the color of the molded product of the present embodiment with the Y.I. (Yellow Index) . The Y.I. of the molded product of the present embodiment is 4 or less, preferably 3.8 or less, and more preferably 3.3 to 3.8, and has an excellent color.

From this, it is possible to suitably use the molded products comprised of the resin as lenses, such as a spectacles lens and a camera lens, as well as optical materials such as a light emitting diode.

In addition, the plastic lens obtained by using the polythiourethane-based resin of the present embodiment may be subjected to physical or chemical treatments such as surface polishing, antistatic treatment, hard coating treatment, non-reflective coating treatment, dyeing treatment, light control treatment in order to impart modifications such as anti-reflection, higher hardness, improved wear resistance, improved chemical resistance, clouding resistance, or a fashionable property, as necessary.

Although the present invention was illustrated using an embodiment, the present invention is not limited to the embodiment described above, and it is possible to adopt various aspects in a range which does not impair the effect of the present invention.

[Examples]

A more detailed description will be given below of the present invention using Examples, but the present invention is not limited thereto.

In the Examples and Comparative Examples below, analysis was performed on the aqueous sodium sulfide solution using the method below.

In addition, the color (APHA) of the obtained "polythiol compound having, as main components, at least one kind selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane" and the color (Y.I.) and the loss degree of transpareancy of the polythiourethane-based molded product obtained by polymerizing the polymerizable composition were evaluated by the test method below.

- Absorbance of sodium sulfide: After dissolving sodium sulfide in degassed water and adjusting to 17.3% by weight, the aqueous solution is added to a quartz cell with an optical path length of 50 mm, and the absorbance at a wavelength of 350 nm was determined using a spectrophotometer (equipment name: UV-1600) manufactured by

Shimadzu Corporation.

Here, as degassed water, water made by blowing nitrogen into water and chasing out dissolved oxygen so as to have a dissolved oxygen concentration of 2 ppm was used.

- Purity: After dissolving the "polythiol compound having, as a main component, at least one kind selected from the group consisting of 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane" in acetonitrile, the sample solution was measured by high performance liquid ion chromatography, and the area percentages were calculated. The liquid ion chromatography device and measurement conditions are as follows.
- Pump: Manufactured by Shimadzu Corporation LC-20AD
- Column: YMC A-312 (ODS) $\phi$6 $\mu$m $\times$ 15 cm
- Mobile phase: Acetonitrile: 0.01 M aqueous solution of $KH_2PO_4$ = 60: 40 (v/v)
- Flow rate: 1.00 ml/min
- Constant temperature bath: Manufactured by Shimadzu Corporation CTO-20A 40°C
- Detector: Manufactured by Shimadzu SPD-20A 230 nm
- Sample preparation: Dissolve 100 mg of polythiol compound in 10 ml of acetonitrile
- Injection amount: 2 $\mu$L
- APHA: A color display method, using a standard solution prepared by dissolving platinum and cobalt reagents, carrying out determination by comparing standard solution dilution solutions of the same depth as the color of the sample, and setting the "frequency" thereof as a measurement value.
- Y.I. (Yellow Index) of polythiourethane-based resin molded product: As an analysis item to evaluate the color of a plastic lens including a polythiourethane-based resin, Y.I. was adopted. A correlation is obtained in which, the smaller the Y.I. value, the better the color of the plastic lens, and the larger the Y.I. value, the worse the color. A circular flat plastic lens having a thickness of 9 mm and a diameter of $\phi$75 mm was created, and chromaticity coordinates x and y were measured using a Coloma-meter CT-210 manufactured by MINOLTA. Based on the x and y value measurement results, the Y.I. was calculated using Formula (1) below.

$$Y.I = (234 * x + 106 * y + 106)/y \quad (1)$$

Here, the Y.I. of the molded product after polymerization finished was set as "Y.I-1" and the Y.I. of the molded product after annealing the obtained molded product under the predetermined conditions was set as "Y.I-2."

- Loss degree of transpareancy: As an analyzing item for evaluating the transparency of the plastic lens containing a polyurethane based resin, the loss degree of transparency was employed. The loss degree of transparency was obtained in the following means. The lens plate of a circular flat plate having a thickness of 9 mm and $\varphi$75 mm was prepared. Then, the lens plate was irradiated with a light source (Luminar Ace LA-150A, a product of Hayashi Watch Works Co., Ltd.) for measuring the loss degree of transparency with a gray scale image processing unit. The captured image was digitized by the gray scale image processing unit to obtain the loss degree of transpareancy.

[Example 1]

(Synthesis of "polythiol compound having, as a main component, at least one kind selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane")

111.58 parts by weight of 99.8% 2-mercaptoethanol, 55.72 parts by weight of degassed water (dissolved oxygen concentration 2 ppm), and 0.78 parts by weight of 30.3% aqueous solution of sodium hydroxide were added into a reactor, and cooled to 10°C. Then, 134.7 parts by weight of 99.94% epichlorohydrin were added dropwise over 3.9 hours at 9 to 11°C, followed by stirring for 60 minutes.

Next, 327.95 parts by weight of 17.3% by weight aqueous sodium sulfide solution having an absorbance at 350 nm of 0.130 were added dropwise at 28 to 30°C over 3.0 hours, and then stirred for 3.0 hours.

Next, 256.4 parts by weight of 99.3% purity thiourea were added thereto, and 170.0 parts by weight of 99.7% hydrochloric acid gas are added thereto at 21 to 73°C while stirring. The temperature was raised in this state, and stirring was carried out for 3 hours under reflux at 110°C to carry out a thiuronium chloride reaction. After cooling to 45°C, 466.2 parts by weight of toluene were added, the result was cooled to 32°C, 340.7 parts by weight of 25.4% by weight aqueous ammonia solution were added thereto at 30 to 38°C over 25 minutes, the temperature was raised to 60°C, then a

hydrolysis reaction was carried out by stirring for 1 hour to obtain a toluene solution of polythiol. 250.0 parts by weight of 4% hydrochloric acid were added to the toluene solution, acid-washing was carried out at 36 to 38°C for 15 minutes, and the wastewater layer was drained, then 125.0 parts by weight of 35% hydrochloric acid were added thereto, and washing of 30 minutes was carried out twice at 35 to 38°C. Then, 125.0 parts by weight of degassed water (dissolved oxygen concentration 2 ppm) were added thereto, and washing of 30 minutes was carried out five times at 35 to 38°C. After removing toluene and a trace amount of water by heating under reduced pressure, 254.1 parts by weight of a polythiol compound having, as a main component, 4,8-dimercaptomethyl-1,11-dimercapto-3,6,9-trithiaundecane and 4,7-dimercaptomethyl-1,11-dimercapto-3,6,9-trithiaundecane and 5,7-dimercaptomethyl-1,11-dimercapto-3,6,9-trithiaundecane was obtained by filtering under reduced pressure with a 1.2 μm PTFE type membrane filter. The analysis results of the obtained polythiol compound are shown in Table 1.

(Manufacture of Plastic Lenses)

50.7 parts by weight of m-xylylene diisocyanate, 0.01 parts by weight of dibutyl tin dichloride as a curing catalyst, 0.10 parts by weight of Zelec UN (product name, product manufactured by Stepan Company; acid phosphate ester), and 0.05 parts by weight of Viosorb 583 (manufactured by Kyodo Chemical Company Limited; UV absorber) was mixed and dissolved at 20°C. 49.3 parts by weight of the obtained polythiol compound having, as a main component, 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane were added and mixed to obtain a uniform mixture liquid. This uniform liquid was degassed at 600 Pa for 1 hour, then filtered with a 3 μm Teflon (registered trademark) filter, and then poured into a mold comprised of glass molds and a tape. The mold was put into an oven, heated gradually from 10°C to 120°C, and polymerized over 20 hours. After the polymerization was completed, the mold was taken out of the oven and released to obtain a molded product. The obtained resin was further annealed at 130°C for 4 hours. The evaluation results of the obtained lens are shown in Table 1.

[Example 2]

254.3 parts by weight of a polythiol compound having, as a main component, 4,8-dimercaptomethyl-1,11-dimercapto-3,6,9-trithiaundecane and 4,7-dimercaptomethyl-1,11-dimercapto-3,6,9-trithiaundecane and 5,7-dimercaptomethyl-1,11-dimercapto-3,6,9-trithiaundecane, were synthesized in the same manner as Example 1 except that, instead of the sodium sulfide used in Example 1, 17.3% aqueous sodium sulfide solution having an absorbance of 0.294 at 350 nm was used. The analysis results of the obtained polythiol compound are shown in Table 1. In addition, a plastic lens was manufactured in the same manner as in Example 1 using the obtained polythiol compound. The evaluation results of the obtained plastic lens are shown in Table 1.

[Example 3]

89.25 parts by weight of 99.8% 2-mercaptoethanol, 44.61 parts by weight of degassed water (dissolved oxygen concentration 2 ppm), and 0.78 parts by weight of 30.3% aqueous solution of sodium hydroxide were added to a reactor and cooled to 10°C. Next, 107.68 parts by weight of 99.94% epichlorohydrin were added dropwise over 4.0 hours at 10 to 11°C, followed by stirring for 60 minutes.

Next, 262.30 parts by weight of 17.3% aqueous solution of sodium sulfide having an absorbance at 350 nm of 0.321 were added dropwise at 27 to 30°C over 3.0 hours, followed by stirring for 3.0 hours.

Next, 214.0 parts by weight of thiourea having a purity of 99.3% were added thereto, and 484.6 parts by weight of 35.0% hydrochloric acid were added thereto while stirring. The temperature was raised in this state, and stirring was carried out for 3 hours under reflux at 110°C to carry out a thiuronium chloride reaction. After cooling to 45°C, 373.0 parts by weight of toluene were added thereto, cooled to 32°C, 347.2 parts by weight of 24.6% by weight aqueous ammonia solution were added thereto at 30 to 38°C over 30 minutes, the temperature was raised to 60°C, then a hydrolysis reaction was carried out while stirring for 1 hour to obtain a toluene solution of polythiol. 130.0 parts by weight of 35% hydrochloric acid were added to the toluene solution, and washing of 30 minutes was carried out twice at 35 to 38°C. Next, 130.0 parts by weight of degassed water (dissolved oxygen concentration 2 ppm) were added thereto, and washing of 30 minutes was carried out five times at 35 to 38°C. After removing toluene and a trace amount of water by heating under reduced pressure, 199.8 parts by weight of a polythiol compound having, as a main component, 4,8-dimercaptomethyl-1,11-dimercapto-3,6,9-trithiaundecane and 4,7-dimercaptomethyl-1,11-dimercapto-3,6,9-trithiaundecane and 5,7-dimercaptomethyl-1,11-dimercapto-3,6,9-trithiaundecane was obtained by filtering under reduced pressure with a 1.2 μm PTFE type membrane filter. The analysis results of the obtained polythiol compound are shown in Table 1. In addition, a plastic lens was manufactured in the same manner as in Example 1 using the obtained polythiol compound. The evaluation results of the obtained plastic lens are shown in Table 1.

[Comparative Example 1]

253.9 parts by weight of a polythiol compound having, as a main component, 4,8-dimercaptomethyl-1,11-dimercapto-3,6,9-trithiaundecane and 4,7-dimercaptomethyl-1,11-dimercapto-3,6,9-trithiaundecane and 5,7-dimercaptomethyl-1,11-dimercapto-3,6,9-trithiaundecane, were synthesized in the same manner as Example 1 except that, instead of the sodium sulfide used in Example 1, 17.3% aqueous sodium sulfide solution having an absorbance of 0.718 at 350 nm was used. The analysis results of the obtained polythiol compound are shown in Table 1. In addition, a plastic lens was manufactured in the same manner as in Example 1 using the obtained polythiol compound. The evaluation results of the obtained plastic lens are shown in Table 1.

[Comparative Example 2]

200.1 parts by weight of a polythiol compound having, as a main component, 4,8-dimercaptomethyl-1,11-dimercapto-3,6,9-trithiaundecane and 4,7-dimercaptomethyl-1,11-dimercapto-3,6,9-trithiaundecane and 5,7-dimercaptomethyl-1,11-dimercapto-3,6,9-trithiaundecane, were synthesized in the same manner as Example 3 except that, instead of the sodium sulfide used in Example 3, 17.3% aqueous sodium sulfide solution having an absorbance of 1.507 at 350 nm was used. The analysis results of the obtained polythiol compound are shown in Table 1. In addition, a plastic lens was manufactured in the same manner as in Example 1 using the obtained polythiol compound. The evaluation results of the obtained plastic lens are shown in Table 1.

[Comparative Example 3]

199.7 parts by weight of a polythiol compound having, as a main component, 4,8-dimercaptomethyl-1,11-dimercapto-3,6,9-trithiaundecane and 4,7-dimercaptomethyl-1,11-dimercapto-3,6,9-trithiaundecane and 5,7-dimercaptomethyl-1,11-dimercapto-3,6,9-trithiaundecane, were synthesized in the same manner as Example 3 except that, instead of the sodium sulfide used in Example 3, 17.3% aqueous sodium sulfide solution having an absorbance of 1.853 at 350 nm was used. The analysis results of the obtained polythiol compound are shown in Table 1. In addition, a plastic lens was manufactured in the same manner as in Example 1 using the obtained polythiol compound. The evaluation results of the obtained plastic lens are shown in Table 1.

Table 1

| | | Example 1 | Example 2 | Example 3 | Comparative Example 1 | Comparative Example 2 | Comparative Example 3 |
|---|---|---|---|---|---|---|---|
| $NaS_2$ quality | absorbance of 17.3% by weight aqueous solution Measuring wavelength: 350 nm | 0.130 | 0.294 | 0.321 | 0.718 | 1.507 | 1.853 |
| Monomer quality | APHA | 10 | 20 | 20 | 25 | 30 | 40 |
| | Purity (area%) | 87.18 | 86.25 | 84.31 | 85.70 | 82.40 | 80.72 |
| Lens quality | Y.I-1 | 3.5 | 3.6 | 3.6 | 4.7 | 4.7 | 5.0 |
| | Y.I-2 | 3.7 | 3.8 | 3.8 | 5.0 | 5.3 | 5.5 |
| | Loss degree of transpareancy | 22 | 23 | 21 | 27 | 23 | 25 |

## Claims

1. A process for producing a polythiol compound, comprising:

reacting a compound represented by following General Formula (3) with sodium sulfide to obtain a polyalcohol compound represented by following Formula (4), and

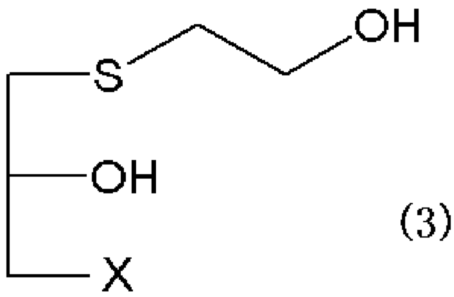

(3)

wherein, in the formula, X represents a halogen atom,

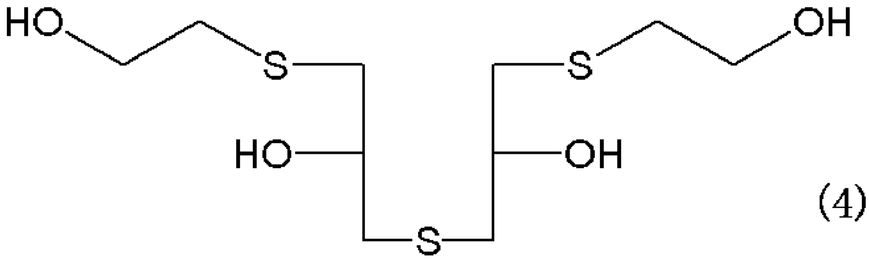

(4)

reacting the obtained polyalcohol compound represented by Formula (4) with a thiating agent to obtain a polythiol compound,
wherein an absorbance of 17.3% by weight aqueous solution of the sodium sulfide at a wavelength of 350 nm, which is measured with a quartz cell with an optical path length of 50 mm, is 0.70 or less.

2. The process for producing a polythiol compound according to claim 1, wherein the absorbance is 0.35 or less.

3. The process for producing a polythiol compound according to claim 1 or 2, further comprising:

reacting 2-mercaptoethanol with an epihalohydrin compound represented by following General Formula (1) to obtain a compound represented by following General Formula (3) before obtaining the polyalcohol compound,

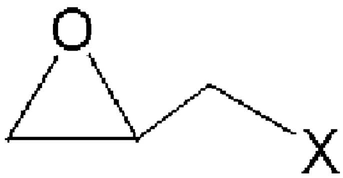

(1)

wherein, in the formula, X represents a halogen atom,

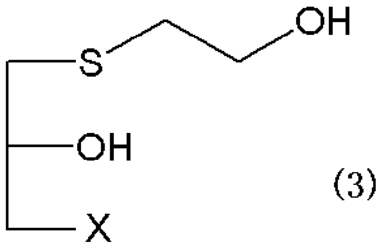

(3)

wherein, in the formula, X represents a halogen atom.

4. The process for producing a polythiol compound according to any one of claims 1 to 3,

wherein reacting the polyalcohol compound with a thiating agent includes
reacting the obtained polyalcohol compound represented by Formula (4) with a thiating agent in a presence of hydrogen chloride to obtain an isothiuronium salt, and
adding ammonia to a reaction solution including the obtained isothiuronium salt and hydrolyzing the isothiuronium salt to obtain a polythiol compound having at least one kind selected from the group consisting of compounds represented by following Formulae (6) to (8) as a main component.

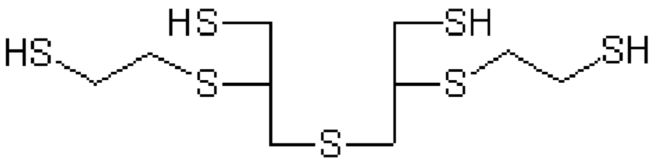

(6)

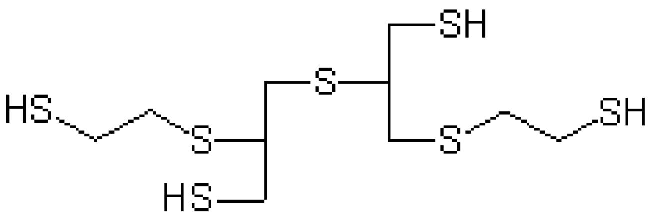

(7)

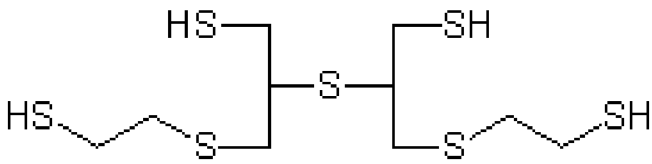

(8)

5. The process for producing a polythiol compound according to any one of claims 1 to 4, wherein the thiating agent is thiourea.

6. The process for producing a polythiol compound according to any one of claims 1 to 5, wherein the absorbance is 0.003 or more.

7. The process for producing a polythiol compound according to any one of claims 1 to 6, wherein X in the General Formula is a chlorine atom.

8. The process for producing a polythiol compound according to any one of claims 1 to 7, further comprising: adding hydrogen chloride to the solution including the obtained polythiol compound and washing to purify the polythiol compound, after reacting the polyalcohol compound with a thiating agent.

9. A polymerizable composition comprising:

the polythiol compound obtained by the manufacturing method according to any one of claims 1 to 8; and a polyiso(thio) cyanate compound.

**10.** A resin obtained by curing the polymerizable composition according to claim 9.

**11.** An optical material comprising:
the resin according to claim 10.

**12.** A lens comprising:
the resin according to claim 10.

**13.** The process for producing a polythiol compound according to claim 1, further comprising:
prior to the step of reacting the compound represented by following General Formula (3) with sodium sulfide, preparing 17.3% by weight aqueous solution of the sodium sulfide and measuring an absorbance at a wavelength of 350 nm of the aqueous solution with a quartz cell with an optical path length of 50 mm to screening the sodium sulfide that the measured absorbance of the aqueous solution is 0.70 or less.

**14.** The process for producing a polythiol compound according to claim 13, wherein the measured absorbance of the aqueous solution is 0.35 or less.

**Patentansprüche**

**1.** Verfahren zur Herstellung einer Polythiol-Verbindung, das Folgendes umfasst:
das Umsetzen einer durch die folgende allgemeine Formel (3) dargestellten Verbindung mit Natriumsulfid, um eine durch die folgende Formel (4) dargestellte Polyalkohol-Verbindung zu erhalten:

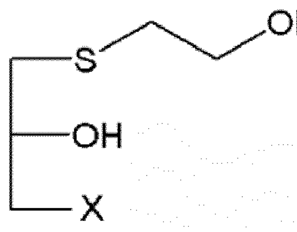

(3)

wobei in der Formel X für ein Halogenatom steht,

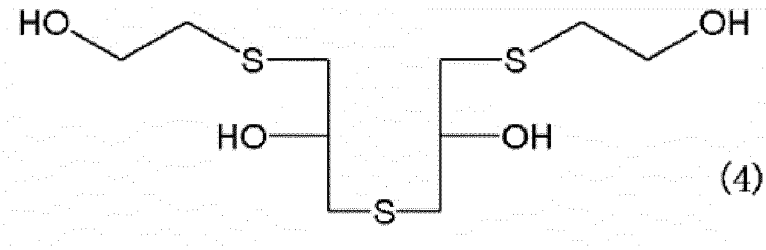

(4)

und das Umsetzen der erhaltenen, durch Formel (4) dargestellten Polyalkohol-Verbindung mit einem Sulfidierungsmittel, um eine Polythiol-Verbindung zu erhalten,
wobei das mit einer Quarzküvette mit einer optischen Weglänge von 50 mm bei einer Wellenlänge von 350 nm gemessene Absorptionsvermögen einer wässrigen Lösung des Natriumsulfids mit 17,3 Gew.-% 0,70 oder weniger beträgt.

**2.** Verfahren zur Herstellung einer Polythiol-Verbindung nach Anspruch 1, wobei das Absorptionsvermögen 0,35 oder weniger beträgt.

**3.** Verfahren zur Herstellung einer Polythiol-Verbindung nach Anspruch 1 oder 2, das weiters Folgendes umfasst:
das Umsetzen von 2-Mercaptoethanol mit einer durch die folgende allgemeine Formel (1) dargestellten Epihalogenhydrin-Verbindung, um eine durch die folgende allgemeine Formel (3) dargestellte Verbindung zu erhalten, bevor die Polyalkohol-Verbindung erhalten wird:

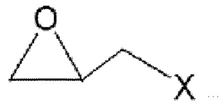 (1)

wobei in der Formel X für ein Halogenatom steht,

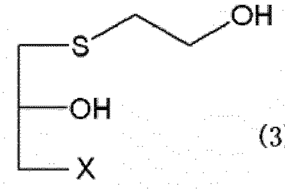 (3)

wobei in der Formel X für ein Halogenatom steht.

**4.** Verfahren zur Herstellung einer Polythiol-Verbindung nach einem der Ansprüche 1 bis 3,
wobei das Umsetzen der Polyalkohol-Verbindung mit dem Sulfidierungsmittel Folgendes umfasst:

das Umsetzen der erhaltenen, durch Formel (4) dargestellten Polyalkohol-Verbindung mit dem Sulfidierungsmittel in der Gegenwart von Chlorwasserstoff, um ein Isothiuroniumsalz zu erhalten, und
das Zusetzen von Ammoniak zu einer Reaktionslösung, die das erhaltene Isothiuroniumsalz enthält, und das Hydrolysieren des Isothiuroniumsalzes, um eine Polythiol-Verbindung zu erhalten, die zumindest eine Verbindung, ausgewählt aus der aus durch die folgenden Formeln (6) bis (8) dargestellten Verbindungen bestehenden Gruppe, als Hauptkomponente umfasst:

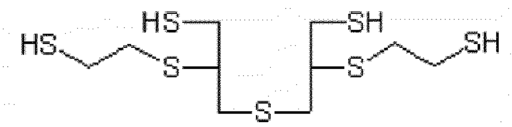 (6)

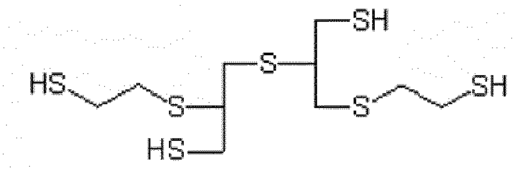 (7)

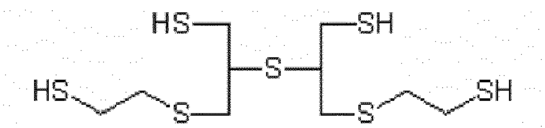 (8)

**5.** Verfahren zur Herstellung einer Polythiol-Verbindung nach einem der Ansprüche 1 bis 4, wobei das Sulfidierungsmittel Thioharnstoff ist.

**6.** Verfahren zur Herstellung einer Polythiol-Verbindung nach einem der Ansprüche 1 bis 5, wobei das Absorptionsvermögen 0,003 oder mehr beträgt.

**7.** Verfahren zur Herstellung einer Polythiol-Verbindung nach einem der Ansprüche 1 bis 6, wobei X in der allgemeinen Formel ein Chloratom ist.

**8.** Verfahren zur Herstellung einer Polythiol-Verbindung nach einem der Ansprüche 1 bis 7, das weiters Folgendes umfasst:
das Zusetzen von Chlorwasserstoff zu der Lösung, welche die erhaltene Polythiol-Verbindung enthält, und Waschen, um die Polythiol-Verbindung zu reinigen, nachdem die Polyalkohol-Verbindung mit dem Sulfidierungsmittel umgesetzt wurde.

**9.** Polymerisierbare Zusammensetzung, die Folgendes umfasst:

eine durch ein Herstellungsverfahren nach einem der Ansprüche 1 bis 8 erhaltene Polythiol-Verbindung; und
eine Polyiso(thio)cyanat-Verbindung.

**10.** Harz, das durch Härten einer polymerisierbaren Zusammensetzung nach Anspruch 9 erhältlich ist.

**11.** Optisches Material, das Folgendes umfasst:
ein Harz nach Anspruch 10.

**12.** Linse, die Folgendes umfasst:
ein Harz nach Anspruch 10.

**13.** Verfahren zur Herstellung einer Polythiol-Verbindung nach Anspruch 1, das weiters Folgendes umfasst:
vor dem Schritt des Umsetzens der durch die folgende allgemeine Formel (3) dargestellten Verbindung mit Natriumsulfid, das Herstellen einer wässrigen Lösung des Natriumsulfids mit 17,3 Gew.-% und das Messen des Absorptionsvermögens der wässrigen Lösung bei einer Wellenlänge von 350 nm mit einer Quarzküvette mit einer optischen Weglänge von 50 mm, um das Natriumsulfid darauf zu untersuchen, dass das gemessene Absorptionsvermögen der wässrigen Lösung 0,70 oder weniger beträgt.

**14.** Verfahren zur Herstellung einer Polythiol-Verbindung nach Anspruch 13, wobei das gemessene Absorptionsvermögen der wässrigen Lösung 0,35 oder weniger beträgt.

## Revendications

**1.** Procédé de production d'un composé polythiol, comprenant les étapes consistant à :

faire réagir un composé représenté par la formule générale (3) suivante avec du sulfure de sodium pour obtenir un composé polyalcool représenté par la formule (4) suivante, et

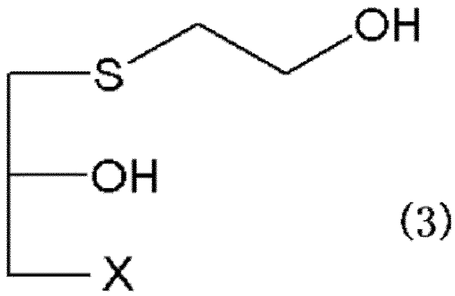

(3)

dans lequel, dans la formule, X représente un atome d'halogène,

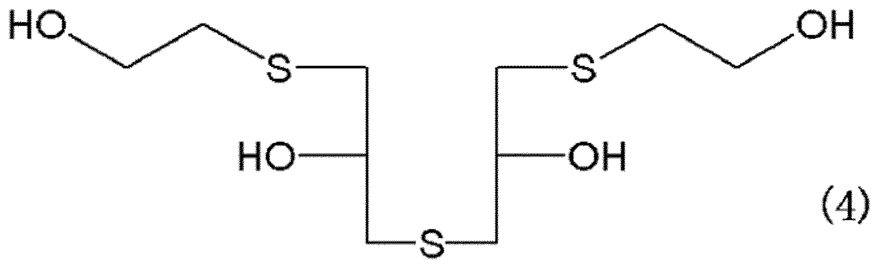

(4)

faire réagir le composé polyalcool obtenu représenté par la formule (4) avec un agent de thiation pour obtenir un composé polythiol,

dans lequel une absorbance d'une solution aqueuse à 17,3 % en poids du sulfure de sodium à une longueur d'onde de 350 nm, qui est mesurée avec une cellule de quartz présentant une longueur de trajet optique de 50 mm, est de 0,70 ou moins.

**2.** Procédé de production d'un composé polythiol selon la revendication 1, dans lequel l'absorbance est de 0,35 ou moins.

**3.** Procédé de production d'un composé polythiol selon la revendication 1 ou 2, comprenant en outre l'étape consistant à :

faire réagir du 2-mercaptoéthanol avec un composé épihalohydrine représenté par la formule générale (1) pour obtenir un composé représenté par la formule générale (3) avant d'obtenir le composé polyalcool,

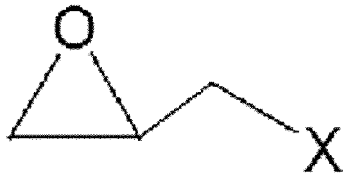

(1)

dans lequel, dans la formule, X représente un atome d'halogène,

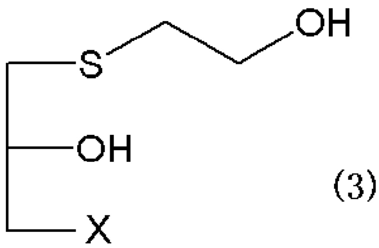

(3)

dans lequel, dans la formule, X représente un atome d'halogène.

**4.** Procédé de production d'un composé polythiol selon l'une quelconque des revendications 1 à 3, dans lequel la réaction du composé polyalcool avec un agent de thiation inclut les étapes consistant à :

faire réagir le composé polyalcool obtenu représenté par la formule (4) avec un agent de thiation en présence de chlorure d'hydrogène pour obtenir un sel d'isothiuronium, et

ajouter de l'ammoniac à une solution de réaction incluant le sel d'isothiuronium obtenu et hydrolyser le sel d'isothiuronium pour obtenir un composé polythiol présentant au moins un type choisi dans le groupe constitué de composés représentés par les formules (6) à (8) suivantes en tant que composant principal

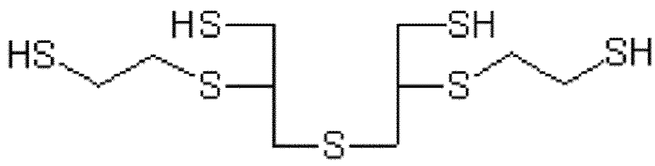

(6)

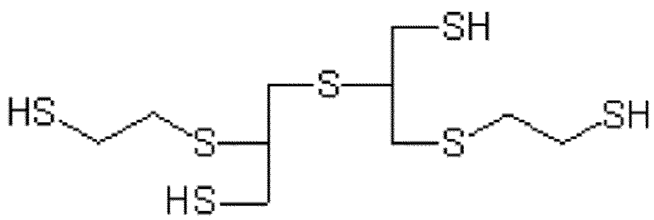

(7)

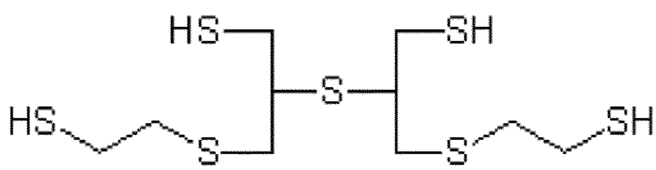

(8)

.

**5.** Procédé de production d'un composé polythiol selon l'une quelconque des revendications 1 à 4, dans lequel l'agent de thiation est la thiourée.

**6.** Procédé de production d'un composé polythiol selon l'une quelconque des revendications 1 à 5, dans lequel l'absorbance est de 0,003 ou plus.

**7.** Procédé de production d'un composé polythiol selon l'une quelconque des revendications 1 à 6, dans lequel X dans la formule générale est un atome de chlore.

**8.** Procédé de production d'un composé polythiol selon l'une quelconque des revendications 1 à 7, comprenant en outre les étapes consistant à :
ajouter du chlorure d'hydrogène à la solution incluant le composé polythiol obtenu et laver pour purifier le composé polythiol, après réaction du composé polyalcool avec un agent de thiation.

**9.** Composition polymérisable, comprenant :

le composé polythiol obtenu par le procédé de fabrication selon l'une quelconque des revendications 1 à 8 ; et un composé polyiso(thio)cyanate.

**10.** Résine obtenu par durcissement de la composition polymérisable selon la revendication 9.

**11.** Matériau optique comprenant :
la résine selon la revendication 10.

**12.** Lentille, comprenant :
la résine selon la revendication 10.

**13.** Procédé de production d'un composé polythiol selon la revendication 1, comprenant en outre les étapes consistant à : avant l'étape consistant à faire réagir le composé représenté par la formule générale (3) suivante avec du sulfure de sodium, préparer une solution aqueuse à 17,3 % en poids du sulfure de sodium et mesurer une absorbance à une longueur d'onde de 350 nm de la solution aqueuse à l'aide d'une cellule de quartz présentant une longueur de trajet optique de 50 mm pour cribler le sulfure de sodium, de sorte que l'absorbance mesurée de la solution aqueuse

soit de 0,70 ou moins.

**14.** Procédé de production d'un composé polythiol selon la revendication 13, dans lequel l'absorbance mesurée de la solution aqueuse est de 0,35 ou moins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H2270859 B
- JP H7252207 B
- WO 2007129449 A
- WO 2007129450 A
- KR 1020120058635
- WO 2016208707 A
- JP 2006284920 A
- EP 2845848 A